# EUROPÄISCHE PATENTSCHRIFT

(11) **EP 0 376 067 B1**
(45) Veröffentlichungstag und Bekanntmachung des Hinweises auf die Patenterteilung: **11.05.1994**
(21) Anmeldenummer: 89123085.6
(22) Anmeldetag: 14.12.1989
(51) Int. Cl.: A61L 15/16, A61K 9/70, A61M 37/00

(54) **Transdermales therapeutisches System mit Physostigmin als wirksamen Bestandteil und Verfahren zu seiner Herstellung**
Transdermal therapeutic system having physostigmine as the active ingredient, and process for its development
Système thérapeutique transdermique pour physostigmine et procédé pour sa préparation

(30) Priorität: 22.12.1988 DE 3843239
(43) Veröffentlichungstag der Anmeldung: 04.07.1990
(73) Patentinhaber: LTS Lohmann Therapie-Systeme GmbH & Co. KG, 56513 Neuwied (DE); Klinge Pharma GmbH, D-81673 München (DE)
(72) Erfinder: Hille, Thomas, Dr., D-5450 Neuwied 1 (DE); Hoffmann, Hans-Rainer, Dr., D-5450 Neuwied 22 (DE); Huber, Hans-Joachim, Dr., D-8000 München 83 (DE); Knoch, Axel, Dr., D-8000 München 80 (DE); Schneider, Gerhard, Dr., D-8011 Baldham (DE); Stanislaus, Fritz, Dr., D-8000 München 80 (DE)
(74) Vertreter: Flaccus, Rolf-Dieter, Dr.

(56) Entgegenhaltungen:
- EP-A- 0 156 080
- EP-A- 0 261 402
- EP-A- 0 305 756
- DE-A- 3 528 979
- DE-A- 3 606 892
- FR-A- 2 497 457
- ADV. BEHAV. BIOL., Band 29 (Alzheimer's Parkinson's Dis.), 1986, pages 557-563; D. LEVY et al.: "A novel transdermal therapeutic system as a potential treatment for Alzheimer's disease"

## Beschreibung

Die Erfindung betrifft ein transdermales therapeutisches System, das Physostigmin als wirksamen Bestandteil enthält und ein Verfahren zu seiner Herstellung.

Die Applikation von Physostigmin zur Behandlung der Alzheimer Krankheit ist in der Literatur beschrieben, wobei die Wirksamkeit der Substanz von verschiedenen Autoren unterschiedlich beurteilt wird. Da das Alkaloid einen hohen first pass effect besitzt - die Bioverfügbarkeit des Physostigmins nach oraler Gabe liegt bei 5 % - müssen die abweichenden Ergebnisse auf Varianten der Applikation zurückgeführt werden.

In der DE-OS 35 28 979 wird eine Zusammensetzung beschrieben, die neben Physostigmin eine Carbonsäure mittlerer Kettenlänge enthält; diese Zusammensetzung kann auf einer Bandage, Einlage oder Kompresse getragen werden, die mittels Verband aufgebracht wird. Diese Applikationsart stellt an sich kein therapeutisches System dar, weshalb vorgesehen ist, die Bandage, Kompresse oder Einlage mit einer Reservoir-Innenschicht einer undurchlässigen Schutzsperrfolie oder einem undurchlässigen Schutzfilm zu versehen und zwischen Reservoir und der Haut eine nicht näher beschriebene Diffusionssteuermembran anzubringen. Weder die Diffusionssteuermembran, noch die Schutzfolien sind näher beschrieben. Die Carbonsäuren werden ausdrücklich als ein wirksames Transportvehikel für die Verabreichung des Arzneimittels durch die Haut bezeichnet, das sonst nicht durch die Hautbarriere hindurchtreten könnte. Diese Aussage ist jedoch wissenschaftlich nicht haltbar.

In der DE-PS 36 06 892 wird eine retardierte Applikation von Physostigmin und anderen Wirkstoffen beschrieben, die transdermal erfolgen kann. Eine spezielle Formulierung wird nicht offenbart, vielmehr wird auf eine bereits beschriebene Formulierung (US-PS 3,921,363) verwiesen.

Neben den nur vagen Ausführungen der transdermalen therapeutischen Systeme wird in keiner der beiden vorstehend erwähnten Offenlegungsschriften auf die Instabilität von Physostigmin eingegangen, die schon früh erkannt wurde (Eber, W., Pharmaz. Ztg 37, 483 (1888); Herzig, J., Mayer, H., Mh.Chem 18, 379 (1897); Herzig J., Lieb, H., ebenda 39, 285 (1918); Solvay, A.A., J. chem. Soc. (London) 101, 978 (1912); Instabilität aufgrund einer raschen Zersetzung setzt dem Einsatz des Physostigmins in der Pharmazie enge Grenzen.

Aufgabe der Erfindung ist daher die Bereitstellung von Physostigmin oder eines seiner pharmazeutisch verträglichen Salze in Form eines transdermalen therapeutischen Systems, das Physostigmin oder dessen pharmazeutisch verträgliches Salz über einen Zeitraum von 24 Stunden kontrolliert abgibt und gewährleistet, daß das Physostigmin sich während der Lagerung des vorgefertigten transdermalen therapeutischen Systems nicht merklich zersetzt.

Diese Aufgabe wird erfindungsgemäß dadurch gelöst, daß das Physostigmin oder eines seiner pharmazeutisch akzeptablen Salze in einer aus besonders ausgewähltem Material bestehenden Reservoirschicht eines transdermalen therapeutischen Systems enthalten ist, wobei die Bestandteile dieser Reservoirschicht, nämlich Polymere, Harze und Weichmacher weder freie Hydroxylgruppen noch Polyethoxygruppen enthalten. Als Harz- und Weichmacherbestandteile der Polymerschicht wurden daher solche aus der Verbindungsklasse der Ester oder Kohlenwasserstoffe ausgewählt.

Die Stabilität des Wirkstoffes kann weiter durch die Wahl eines geeigneten Lösemittels bzw. Lösemittelgemisches bei der Herstellung des transdermalen therapeutischen Systems verbessert werden. Hier werden bevorzugt Lösemittel bzw. Lösemittelgemische eingesetzt, die bei niedrigem Siedepunkt und dadurch schonender Trocknung die Erzielung eines äußerst geringen Lösemittelrestgehalts von kleiner als 0,5, vorzugsweise kleiner als 0,4% ermöglichen.

Gegenstand der Erfindung ist somit ein transdermales therapeutisches System zur Verabreichung von Physostigmin an die Haut aus einer wirkstoffundurchlässigen Deckschicht, einer haftklebenden Reservoirschicht und gegebenenfalls einer wieder ablösbaren Schutzschicht, dessen Reservoirschicht ausgehend von einer Lösung in niedrigsiedendem Lösemittel oder Lösemittelgemisch folgende Anteile in homogener Feinverteilung enthält:
- 10 bis 90 Gew.-% Polymermaterial, ausgewählt aus den Gruppen, bestehend aus Blockcopolymeren auf Basis von Styrol und 1,3-Dienen, Polyisobutylen, Polymeren auf Acrylat- und/oder Methacrylatbasis, und gegebenenfalls als Zusätze Ester hydrierten Kolophoniums,
- 0 bis 30 Gew.-% Weichmacher auf Basis von Kohlenwasserstoffen und/oder Estern, und
- 0,1 bis 20 Gew.-% Physostigmin.

Dabei kann die wirkstoffundurchlässige Deckschicht aus flexiblem oder nicht flexiblem Material bestehen. Substanzen, die zu ihrer Herstellung verwendet werden können, sind Polymerfolien oder Metallfolien, wie Aluminiumfolie, die allein oder mit einem polymeren Substrat beschichtet, angewandt werden. Es können auch textile Flächengebilde verwendet werden, wenn die Bestandteile des Reservoirs aufgrund ihrer physikalischen Beschaffenheit durch sie nicht hindurch treten können. Bei einer bevorzugten Ausführungsform ist die Deckschicht ein Verbundstoff, aus einer mit Aluminium bedampften Folie.

Die Reservoirschicht besteht aus einer Polymermatrix und dem Wirkstoff, wobei die Polymermatrix die Eigenschaft besitzt, den Zusammenhalt des Systems zu gewährleisten. Sie besteht aus einem Grundpolymer und gegebenenfalls den üblichen Zusätzen. Die Auswahl des Grundpolymers richtet sich nach den chemischen und physikalischen Eigenschaften des Physostigmins. Beispielhafte Polymere sind Kautschuk, kautschukähnliche, synthetische Homo-, Co- oder Blockpolymere, Polyacrylsäureester und deren Copolymere. Grundsätzlich kommen alle Polymere in Frage, die bei der Herstellung von Haftklebern eingesetzt werden, physiologisch unbedenklich sind und Physostigmin nicht zersetzen. Besonders bevorzugt sind solche, die aus Blockcopolymeren auf Basis von Styrol und 1,3-Dienen, Polyisobutylenen oder Polymeren aus Acrylat- und/oder Methacrylat bestehen. Von den Blockcopolymeren auf Basis von Styrol und 1,3-Dienen werden ganz besonders lineare Styrol-Isopren-Blockcopolymere eingesetzt.

Als Polymere auf Acrylatbasis werden Acrylatcopolymere aus 2-Ethylhexylacrylat, Vinylacetat und Acrylsäure mit bzw. ohne Titanchelatester bevorzugt. Als Methacrylate werden Copolymere auf Basis von Dimethylaminoethylmethacrylate und neutralen Methacrylsäureestern bevorzugt. Als Zusätze werden Ester von hydriertem Kolophonium, vorzugsweise insbesondere dessen Methyl- und Glycerinester verwendet.

Die Art der möglichen übrigen Zusätze hängt vom eingesetzten Polymer und dem Wirkstoff ab: Nach ihrer Funktion lassen sie sich einteilen in Weichmacher, Klebrigmacher, Stabilisatoren, Trägerstoffe, diffusions- und penetrationsregulierende Zusätze oder Füllstoffe. Die hierfür in Frage kommenden physiologisch unbedenklichen Substanzen sind dem Fachmann bekannt. Die Reservoirschicht besitzt eine solche Eigenklebrigkeit, daß ein dauernder Kontakt zur Haut sichergestellt ist.

Beispiele für geeignete Weichmacher sind Diester von Dicarbonsäure, z.B. Di-n-butyladipat sowie Triglyceride, insbesondere mittelkettige Triglyceride der Capryl/Caprinsäure des Kokosöls zu nennen. Weitere Beispiele für einen geeigneten Weichmacher sind Isopropylmyristat, Dioctylcyclohexan u.a.

Die ablösbare Schutzschicht, die mit der Reservoirschicht in Berührung steht, und vor der Anwendung entfernt wird, besteht beispielsweise aus den selben Materialien, wie sie zur Herstellung der Deckschicht benutzt werden, vorausgesetzt, daß sie ablösbar gemacht werden, wie z.B. durch eine Silikonbehandlung. Andere ablösbare Schutzschichten sind z.B. Polytetrafluorethylen, behandeltes Papier, Cellophan, Polyvinylchlorid u.ä. Wird das erfindungsgemäße Laminat vor Aufbringen der Schutzschicht in therapiegerechte Formate (Pflaster) aufgeteilt, so können die dann aufzubringenden Schutzschichtformate ein überstehendes Ende aufweisen, mit dessen Hilfe sie leichter von dem Pflaster abgezogen werden können.

Das erfindungsgemäße transdermale therapeutische System wird hergestellt, indem der Wirkstoff zusammen mit den Bestandteilen der haftklebenden Reservoirschicht gegebenenfalls in Lösung homogen vermischt und auf die wirkstoffundurchlässige Deckschicht aufgestrichen wird, worauf gegebenenfalls das Lösemittel oder die Lösemittel entfernt wird/werden. Anschließend wird die Klebeschicht mit einer entsprechenden Schutzschicht versehen.

Die Erfindung wird durch die folgenden Beispiele erläutert:

### Beispiel 1:

20 g n-Heptan und 80 g Methylethylketon werden gemischt. In 90 g dieser Mischung löst man 7,2 g Physostigmin, freie Base. Nach vollständiger Auflösung des Wirkstoffes gibt man portionsweise 40 g eines Glycerinesters von völlig hydriertem Kolophonium und portionsweise 40,0 g eines linearen Styrol-Isopren-Styrol-Blockcopolymers sowie 5,6 g Triglyceride der Capryl/Caprinsäuren des Kokosöls ("Mittelkettige Triglyceride", DAB 8) zu. Unter Lichtausschluß rührt man bei Raumtemperatur 8 Stunden lang bis zur vollständigen Auflösung und streicht die erhaltene Lösung mit einem 250 µm Rakel auf eine aluminisierte und silikonisierte Polyethylen-Folie.

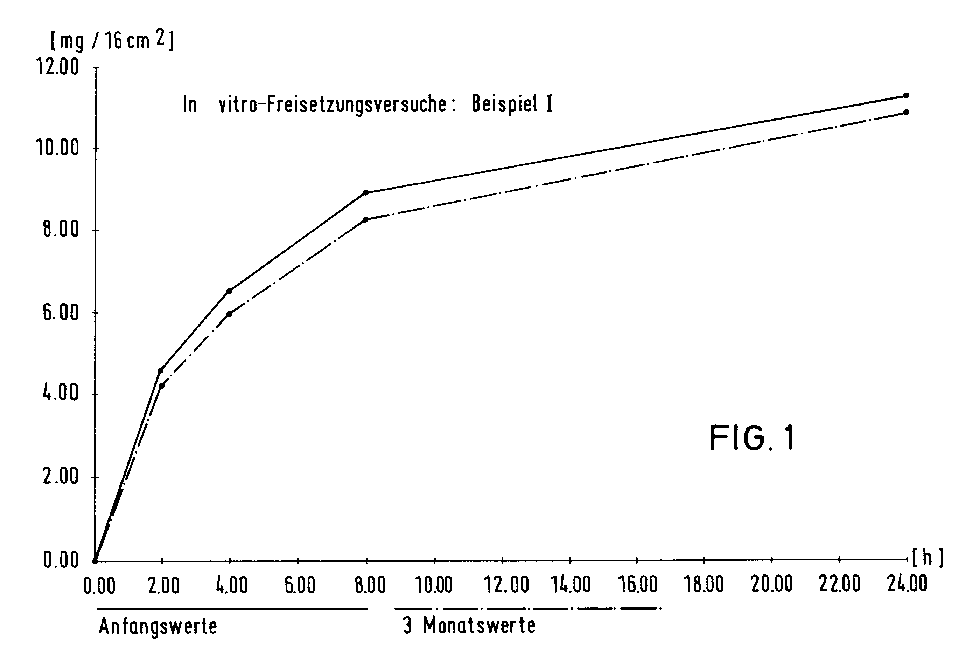

Nachdem das Lösemittel durch 25 minütiges Trocknen bei 50°C entfernt wurde, deckt man den Klebefilm mit einer Polyesterfolie 15 µ ab. Mit geeigneten Schneidewerkzeugen stanzt man eine Fläche von 16 cm² aus und entfernt die Ränder durch Abgittern. Die Freisetzungsgraphiken des Rezepturbeispiels 1 sind in den Figuren 1 - 2 wiedergegeben. Die Graphiken zeigen die kontrollierte Freisetzung des Wirkstoffs sowohl in eine physiologische Kochsalz-Lösung als auch durch exzidierte Nagetierhaut.

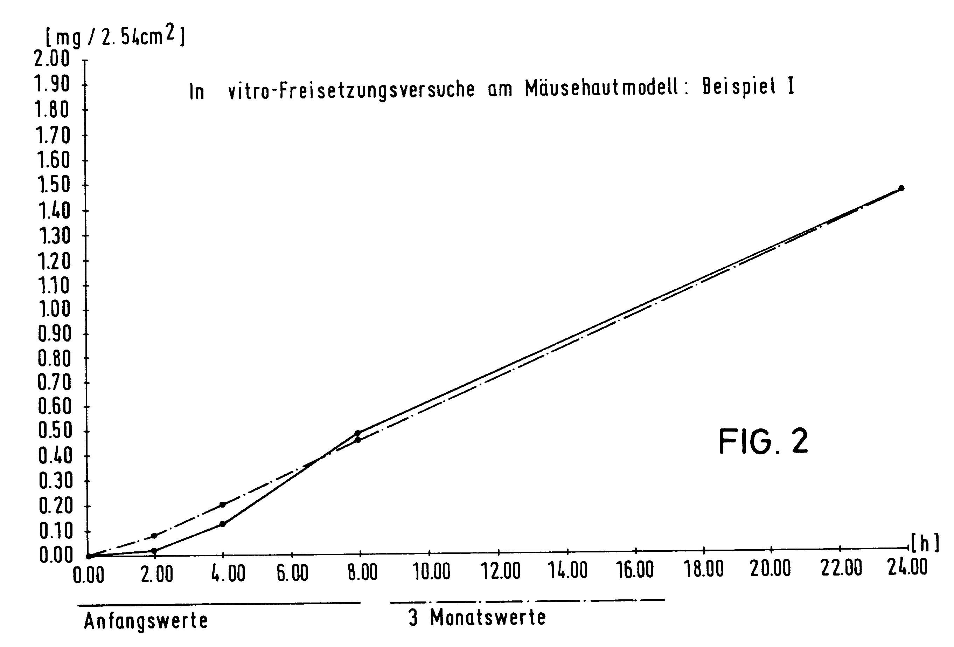

Dabei stammt die Kurve in Figur 1, deren Linie nicht unterbrochen ist, von in-vitro-Freisetzungen durch Muster, die unmittelbar nach ihrer Fertigung untersucht wurden. Die unterbrochene Kurve wurde erhalten durch Freisetzung durch Mustern nach dreimonatiger Lagerung bei Raumtemperatur. Da beide Kurven annähernd deckungsgleich sind, kann die oben erwähnte Stabilität eindrucksvoll demonstriert werden. Figur 2 zeigt, daß die Penetrationsrate von Mustern, die direkt nach Fertigung bzw. nach dreimonatiger Lagerung untersucht wurden, ebenfalls annähernd gleich sind.

Die Stabilität des Wirkstoffes im System wurde aber auch noch durch Gehaltsbestimmungen direkt nach der Fertigung bzw. nach dreimonatiger Lagerung aufgezeigt. Dabei konnten weder die aus der Literatur bekannten Abbauprodukte Eserolin und Rubreserin noch andere bisher nicht beschriebene detektiert werden. Dazu wurde folgende Methode verwandt:

### Probenvorbereitung:

1 Pflaster mit Abdeckfolie wird mittels Schere geviertelt, die Abdeckfolie entfernt und zusammen mit den Pflasterteilen in einem verschließbaren, lichtgeschützten Glasgefäß mit 50,0 ml Tetrahydrofuran (p.a.) mindestens 2 Stunden geschüttelt, 10 Minuten ultrabeschallt und anschließend zentrifugiert. Verdünnung für HPLC mit Methanol und nochmalige Zentrifugation.

Anschließend wird der Gehalt des Physostigmins im Zentrifugat per HPLC bestimmt.

### Beispiel 2:

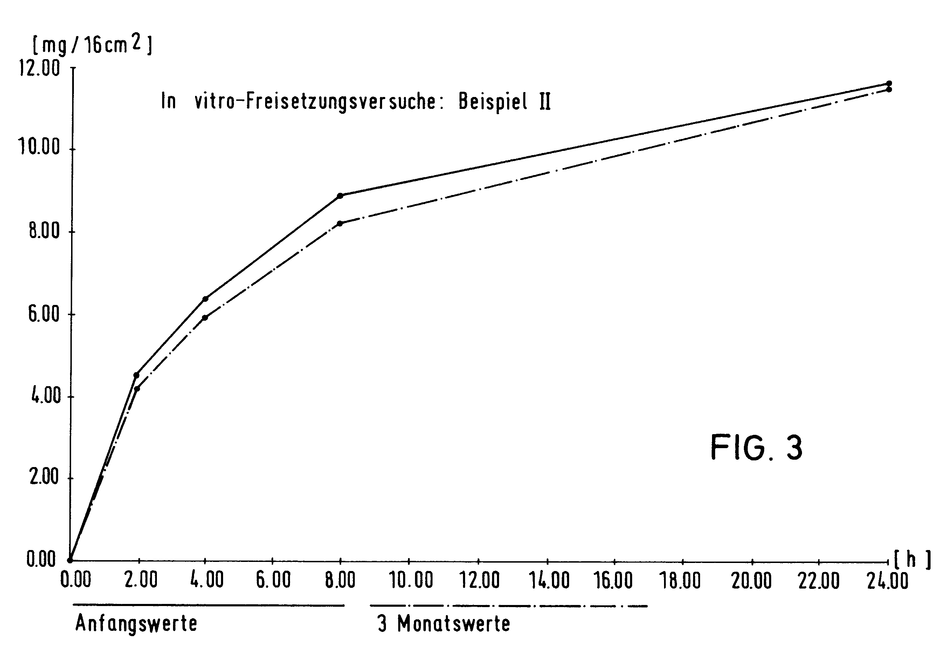

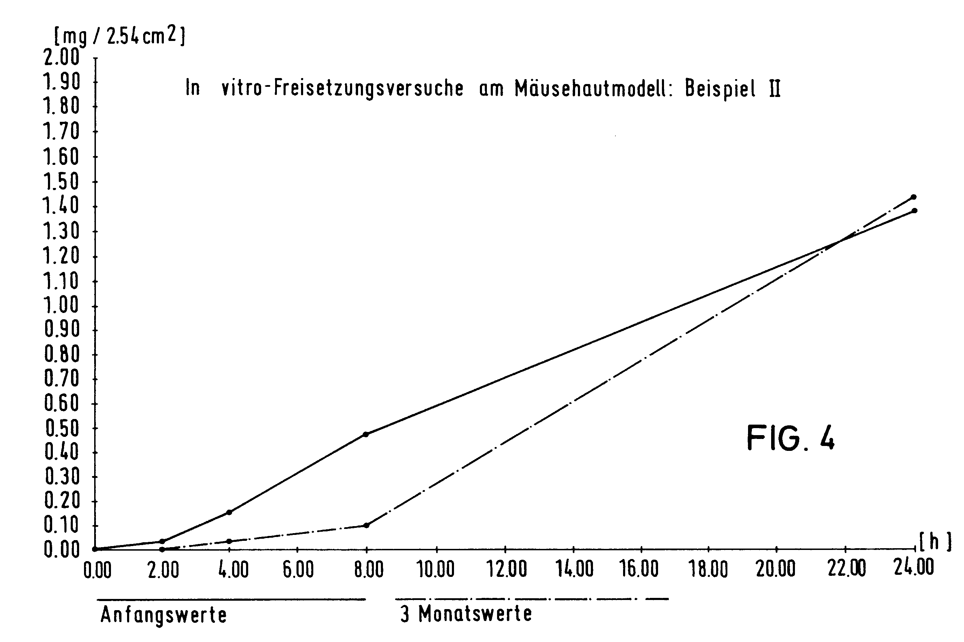

Die Ausführung erfolgt wie beim Beispiel 1 mit dem Unterschied, daß statt 5,6 g Triglyceriden der Capryl/Caprinsäuren 3,2 g Di-n-butyladipat eingesetzt werden. Die Freisetzungsgraphiken des Rezepturbeispiels 2 sind in den Figuren 3-4 wiedergegeben. Die Graphiken zeigen die kontrollierte Freisetzung des Wirkstoffes sowohl in eine physiologische Kochsalz-Lösung als auch durch exidierte Nagetierhaut. Wie bei Beispiel 1 zeigt die Kurve mit der durchgehenden Linie die Freisetzung der Muster direkt nach der Fertigung. Die unterbrochene Kurve stammt von Freisetzungen von Mustern, die drei Monate bei Raumtemperatur gelagert wurden. Auch bei diesen Mustern, laufen die Kurven fast deckungsgleich, so daß auch bei diesem Beispiel stabile Pflaster erhalten wurden.

Wie in Beispiel 1 wurde auch der Gehalt an Physostigmin bestimmt, ohne daß ein Abbauprodukt nach dreimonatiger Lagerung nachgewiesen werden konnte.

### Beispiel 3:

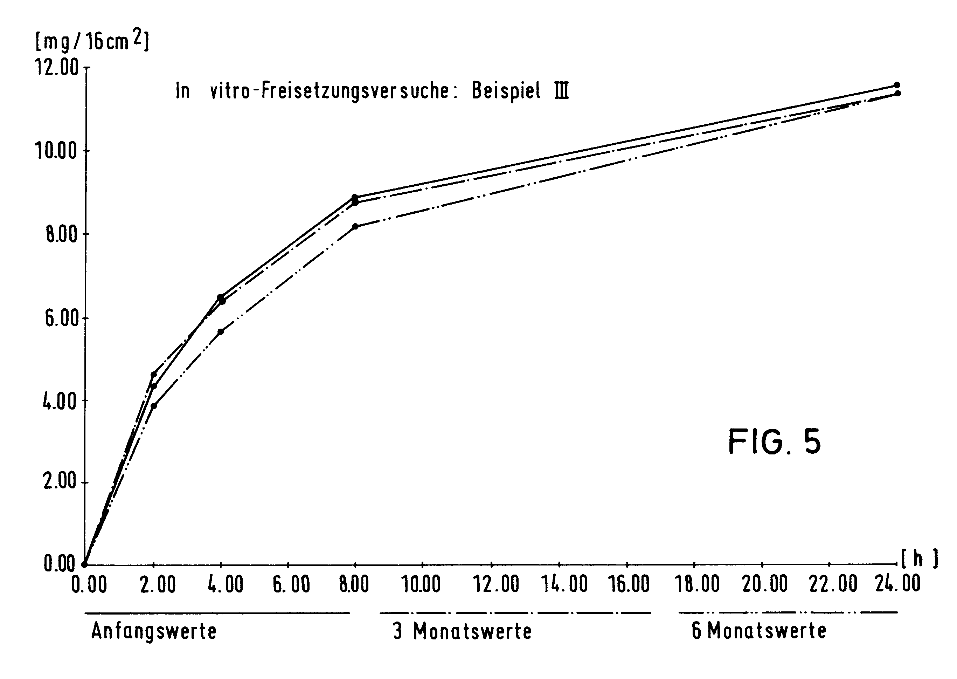

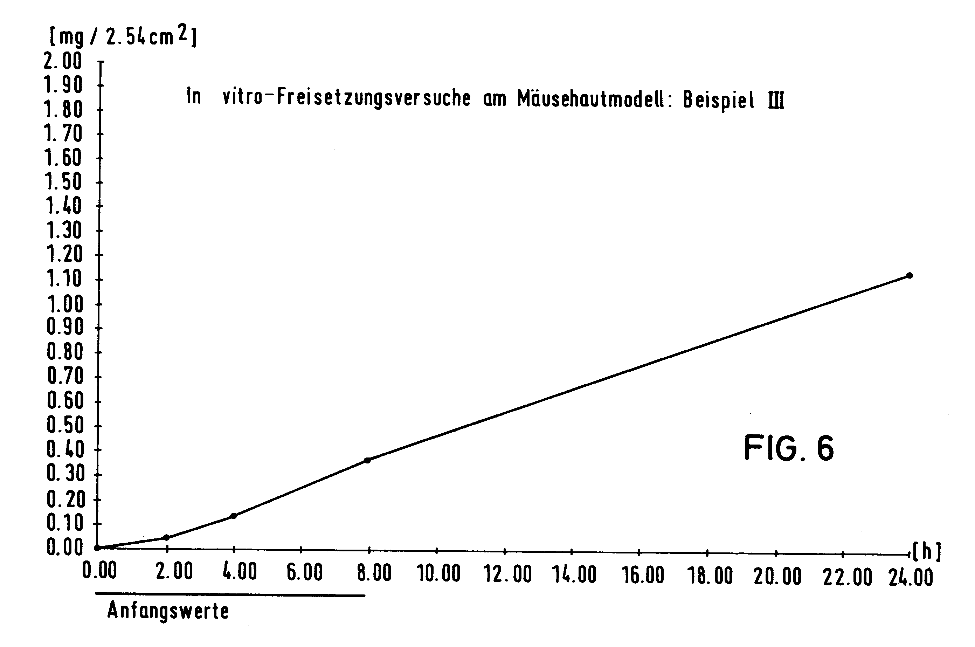

2,0 g Physostigmin, freie Base, werden in einen Kolben eingewogen. Unter Rühren gibt man 25 g einer 60 %igen Lösung von Glycerinkolophonium-Ester in Butanon und 25 g einer 40%igen Lösung eines Styrol-Butadien-Blockcopolymer, in einer Mischung aus n-Heptan und Butanon im Verhältnis 1: 2 hinzu. Nach inniger Vermischung werden unter Rühren noch 2,5 g Methylester von hydriertem Kolophonium, und 1,95 g Triglyceride von Capryl/Caprinsäuren zugesetzt. Die weitere Ausführung erfolgt wie unter Beispiel 1 beschrieben, die Freisetzungsgraphiken sind in den Figuren 5-6 dargestellt. Die Graphiken zeigen die kontrollierte Freisetzung des Wirkstoffs sowohl in eine physiologische Kochsalz-Lösung als auch durch exidierte Nagetierhaut.

Wie bei Beispiel 1 und 2 zeigt die Kurve mit der durchgehenden Linie die Freisetzung der Muster direkt nach Fertigung. Anders aber als in den vorangestellten Mustern wurde die Freisetzung nicht nur nach dreimonatiger, sondern nach sechsmonatiger Lagerung bestimmt. Alle drei Kurven laufen wieder annähernd deckungsgleich, so daß auch nach einem halben Jahr Lagerzeit die gleiche Freisetzung erzielt wird wie direkt nach der Fertigung.

Wie in den Beispielen 1 und 2 konnte per HPLC kein Abbauprodukt nach sechsmonatiger Lagerung nachgewiesen werden.

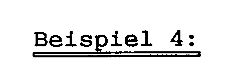

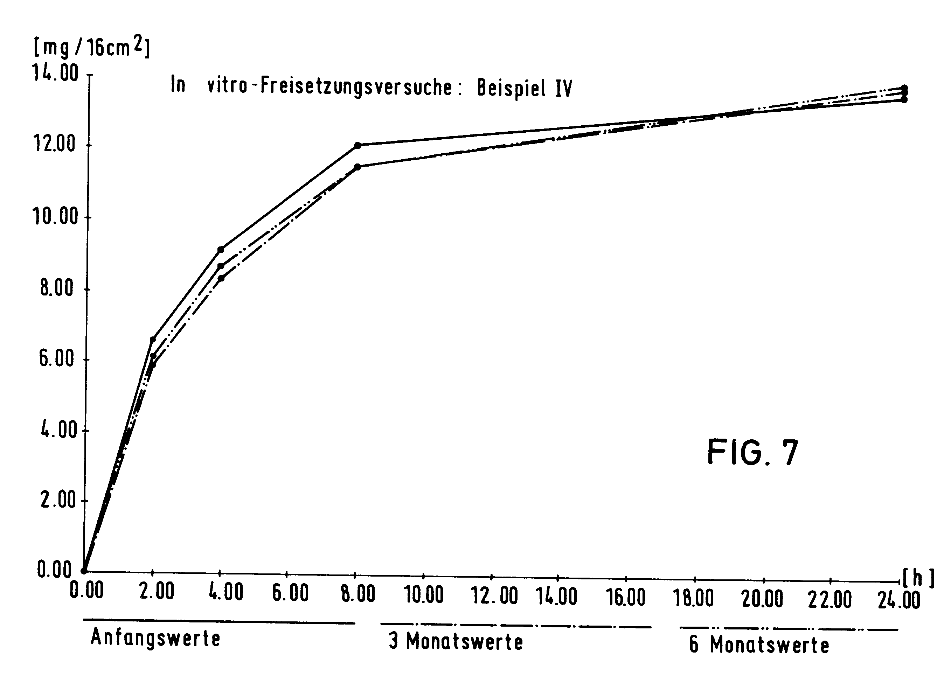

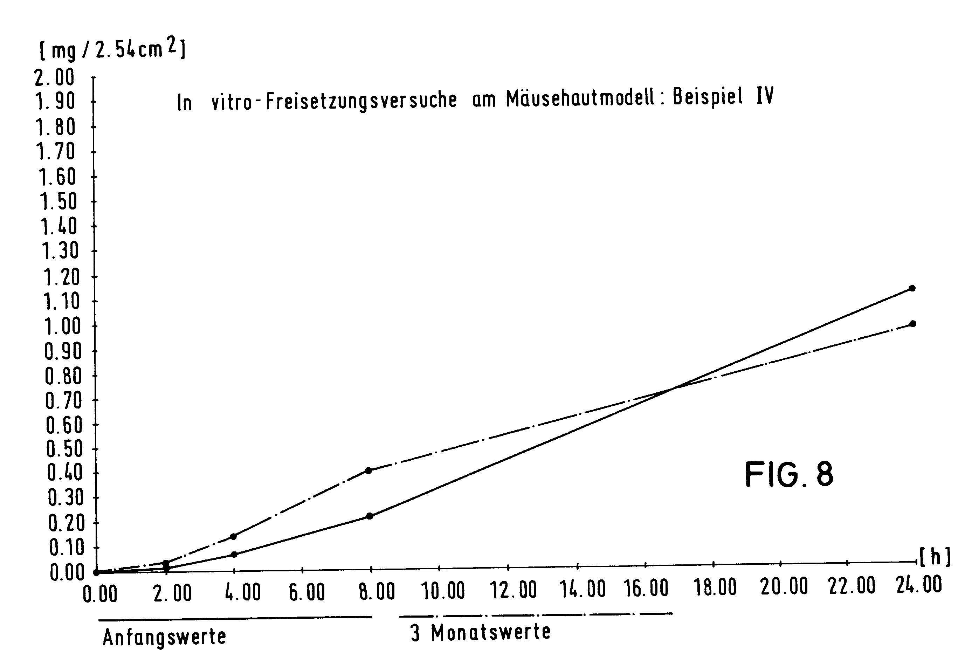

8,5 g Physostigmin, freie Base, werden zusammen mit 21,3 g eines kationischen Copolymerisats auf Basis Dimethylaminoethylmethacrylat und neutralen Methacrylsäureestern in 21,4 g Ethylacetat gelöst. Unter Rühren werden 8,5 Triglyceride der Capryl/Caprinsäuren, und 68,3 g eines nicht selbstvernetzendes Acrylatcopolymers aus 2-Ethylhexylacrylat, Vinylacetat und Acrylsäure (50 %ig in Ethylacetat) zugegeben. Nach höchstens halbstündigem Rühren bei Raumtemperatur ist die Klebemasse homogen. Die weitere Bearbeitung erfolgt wie unter Beispiel 1 beschrieben. Die Freisetzungsdaten sind in den Figuren 7 - 8 wiedergegeben. Die Graphiken zeigen die kontrollierte Freisetzung des Wirkstoffs sowohl in eine physiologische Kochsalz-Lösung als auch durch exzidierte Nagetierhaut.

Wie in den vorangegangenen Beispielen zeigt die Kurve mit der durchgehenden Linie die Freisetzung der Muster direkt nach der Fertigung. Anders aber als in den vorangestellten Mustern wurde die Freisetzung nicht nur nach dreimonatiger, sondern auch nach sechsmonatiger Lagerung bestimmt. Alle drei Kurven laufen wieder annähernd deckungsgleich, so daß auch nach einem halben Jahr Lagerzeit die gleiche Freisetzung erzielt wird, wie direkt nach der Fertigung.

Wie in den vorangegangenen Beispielen konnte mit der bei Beispiel 1 beschriebenen HPLC-Methode kein Abbauprodukt nach sechsmonatiger Lagerung erfasst werden.

### Beispiel 5:

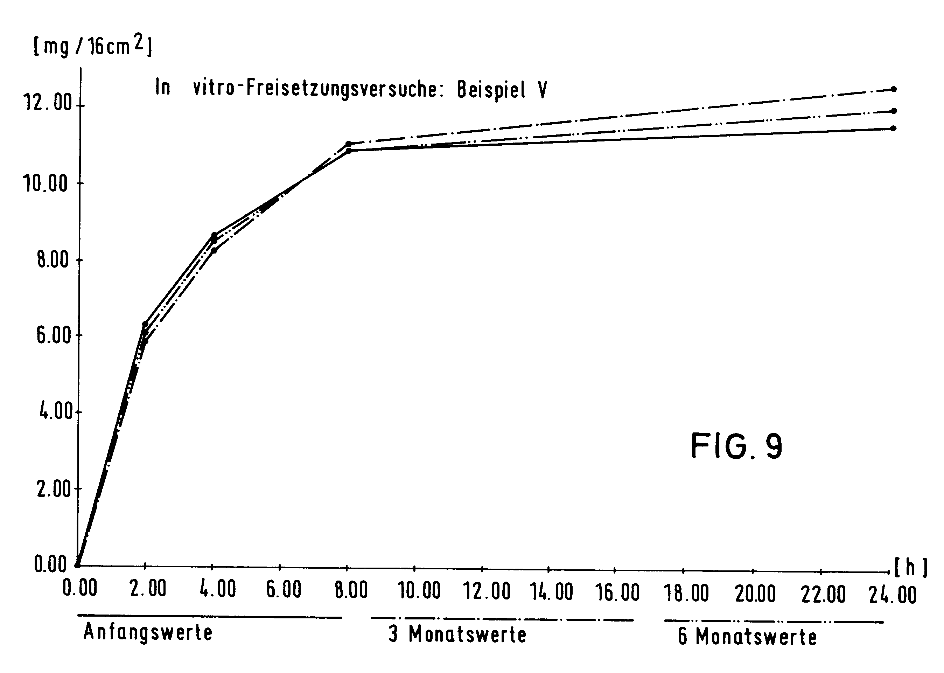

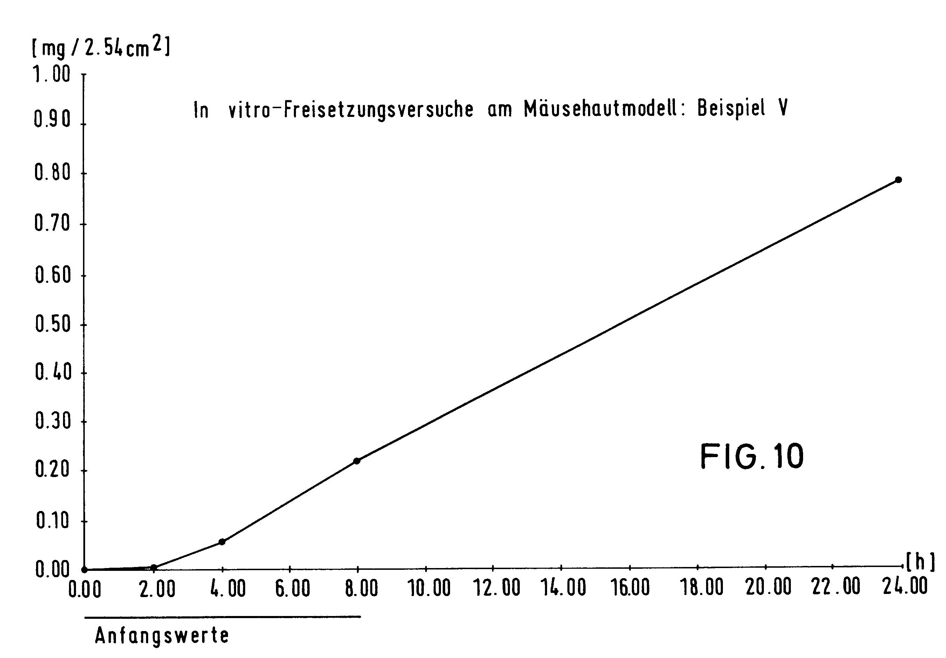

Die Ausführung erfolgt wie in Beispiel 4 beschrieben mit dem Unterschied, daß das Acrylatcopolymer nicht 50 %ig in Ethylacetat, sondern 40 %ig in einem Lösungsmittelgemisch (Ethylacetat: Ethanol: Heptan: Methanol 64:25:9:2) gelöst ist und einen Vernetzer enthält. Die Freisetzungsgraphiken sind in den Figuren 9 - 10 wiedergegeben.

Wie in den vorangegangenen Beispielen zeigt die Kurve mit der durchgehenden Linie die Freisetzung der Muster direkt nach der Fertigung. Anders aber als in den vorangestellten Mustern wurde die Freisetzung nicht nur nach dreimonatiger, sondern auch nach sechsmonatiger Lagerung bestimmt. Alle drei Kurven laufen wieder annähernd deckungsgleich, so daß auch nach einem halben Jahr Lagerzeit die gleiche Freisetzung erzielt wird wie direkt nach der Fertigung.

Anzumerken ist bei Beispiel 5 noch, daß Physostigmin zwar einem Lösemittel - nämlich Ethanol - ausgesetzt wird, das diesen Wirkstoff hydrolytisch spalten kann (Pfeifer, S.; Behnsen, G. und Kühn., L., Pharmazie 27, 639 (1972); entscheidend ist aber, daß dies nur kurz und unter Lichtausschluß einwirkt, da es nach dem Ausstreichen durch schonende Trocknung restlos entfernt wird. Aus schon dargelegten Gründen greifen weder das Grundpolymer, noch das Hartharz oder der Weichmacher den Wirkstoff an.

Für die Stabilität des Wirkstoffes ist entscheidend, daß die eingesetzten Polymere, Harze und Weichmacher weder freie Hydroxylgruppen noch Polyethoxygruppen enthalten, da der Anteil an Wirkstoff, der gelöst vorliegt, sonst der Hydrolyse unterliegen würde. Daher wurden Harze und Weichmacher der Verbindungsklasse Ester gewählt.

Für die Stabilität des Wirkstoffes ist auch die Wahl des Lösemittels bzw. des Lösemittelgemisches entscheidend. Wenn dieses vor der Trocknung über Stunden auf Physostigin einwirkt. Der Anteil des zur Unterdrückung der Blasenbildung gegebenenfalls erforderlichen höher siedenden Lösemittels muß gering sein. Dies wird in der vorliegenden Erfindung bei den Beispielen 1 bis 3 dadurch erreicht, daß eine Mischung aus Butanon und n-Heptan gewählt wurde, die ein azeotropes Gemisch (Verhältnis Butanon : n-Heptan 70 : 30, Siedepunkt: 77°C; Siedepunkt Butanon: 79,6°C, Siedepunkt n-Heptan: 98,5°C bilden. Dadurch kann trotz schonender Trocknung ein maximaler Lösemittelrestgehalt kleiner als 0,4% erreicht werden.

Da Polyacrylate nicht zur Blasenbildung neigen, war dieses Vorgehen bei den Beispielen 4 und 5 nicht erforderlich.

## Patentansprüche (Patentansprüche für folgende(n) Vertragsstaat(en): AT, BE, CH, DE, FR, GB, IT, LI, LU, NL, SE)

1. Transdermales therapeutisches System zur Verabreichung von Physostigmin an die Haut aus einer wirkstoffundurchlässigen Deckschicht, einer haftklebenden Reservoirschicht und gegebenenfalls einer wiederablösbaren Schutzschicht, dadurch gekennzeichnet, daß die Reservoirschicht ausgehend von einer Lösung in niedrig siedendem Lösemittel oder Lösemittelgemisch folgende Anteile in homogener Feinverteilung enthält:
- 10 bis 90 Gew.-% Polymermaterial, ausgewählt aus den Gruppen, bestehend aus Blockcopolymeren auf Basis von Styrol und 1,3-Dienen, Polyisobutylen, Polymeren auf Acrylat- und/oder Methacrylatbasis, und gegebenenfalls als Zusätze Ester hydrierten Kolophoniums,
- 0 bis 30 Gew.-% Weichmacher auf Basis von Kohlenwasserstoffen und/oder Estern, und
- 0,1 bis 20 Gew.-% Physostigmin

2. Transdermales therapeutisches System nach Anspruch 1, dadurch gekennzeichnet, daß das Polymermaterial lineares Styrol-Isopren-Styrol-Blockcopolymeres enthält.

3. Transdermales therapeutisches System nach Anspruch 1, dadurch gekennzeichnet, daß das Polymermaterial lineares Styrol-Butadien-Styrol-Blockcopolymeres enthält.

4. Transdermales therapeutisches System nach Anspruch 1, dadurch gekennzeichnet, daß das Polymermaterial selbstvernetzendes Acrylatcopolymeres aus 2-Ethylhexylacrylat, Vinylacetat, Acrylsäure und Titanchelatester enthält.

5. Transdermales therapeutisches System nach Anspruch 1, dadurch gekennzeichnet, daß das Polymermaterial nicht selbstvernetzendes Acrylatcopolymere aus 2-Ethylhexylacrylat, Vinylacetat und Acrylsäure enthält.

6. Transdermales therapeutisches System nach Anspruch 1, dadurch gekennzeichnet, daß das Polymermaterial als Polymer auf Basis von Methacrylaten ein Copolymer auf Basis von Dimethylaminoethylmethacrylat und neutralen Methacrylsäureestern enthält.

7. Transdermales therapeutisches System nach Anspruch 1, dadurch gekennzeichnet, daß das Polymermaterial als Ester des hydrierten Kolophoniums dessen Methylester enthält.

8. Transdermales therapeutisches System nach Anspruch 1, dadurch gekennzeichnet, daß das Polymermaterial als Ester des hydrierten Kolophoniums dessen Glycerinester enthält.

9. Transdermales therapeutisches System nach Anspruch 1, dadurch gekennzeichnet, daß die Reservoirschicht als Weichmacher Dioctylcyclohexan enthält.

10. Transdermales therapeutisches System nach Anspruch 1, dadurch gekennzeichnet, daß die Reservoirschicht als Weichmacher Di-n-butyladipat enthält.

11. Transdermales therapeutisches System nach Anspruch 1, dadurch gekennzeichnet, daß die Reservoirschicht als Weichmacher Triglyceride enthält.

12. Transdermales therapeutisches System nach Anspruch 1, dadurch gekennzeichnet, daß die Reservoirschicht als Weichmacher Isopropylmyristat enthält.

13. Verfahren zur Herstellung eines transdermalen therapeutischen Systems nach einem der Ansprüche 1 bis 12, dadurch gekennzeichnet, daß die Bestandteile der Reservoirschicht in einem niedrig siedenden Lösemittel gelöst werden, das eine Trocknung bis zu einem maximalen Lösemittelrestgehalt von kleiner 0,4 Gew.-% ermöglicht.

14. Verfahren zur Herstellung eines transdermalen therapeutischen Systems nach Anspruch 13, dadurch gekennzeichnet, daß man ein Lösemittelgemisch aus Butanon und n-Heptan verwendet.

## Patentansprüche (Patentansprüche für folgende(n) Vertragsstaat(en): ES, GR)

1. Verfahren zur Herstellung eines transdermalen therapeutischen Systems zur Verabreichung von Physostigmin an die Haut aus einer wirkstoffundurchlässigen Deckschicht, einer haftklebenden Reservoirschicht und gegebenenfalls einer wiederablösbaren Schutzschicht, dadurch gekennzeichnet, daß die Reservoirschicht ausgehend von einer Lösung in niedrig siedendem Lösemittel oder Lösemittelgemisch folgende Anteile in homogener Feinverteilung enthält:
- 10 bis 90 Gew.-% Polymermaterial, ausgewählt aus den Gruppen, bestehend aus Blockcopolymeren auf Basis von Styrol und 1,3-Dienen, Polyisobutylen, Polymeren auf Acrylat- und/oder Methacrylatbasis, und gegebenenfalls als Zusätze Ester hydrierten Kolophoniums,
- 0 bis 30 Gew.-% Weichmacher auf Basis von Kohlenwasserstoffen und/oder Estern, und
- 0,1 bis 20 Gew.-% Physostigmin

2. Verfahren nach Anspruch 1, dadurch gekennzeichnet, daß das Polymermaterial lineares Styrol-Isopren-Styrol-Blockcopolymeres enthält.

3. Verfahren nach Anspruch 1, dadurch gekennzeichnet, daß das Polymermaterial lineares Styrol-Butadien-Styrol-Blockcopolymeres enthält.

4. Verfahren nach Anspruch 1, dadurch gekennzeichnet, daß das Polymermaterial selbstvernetzendes Acrylatcopolymeres aus 2-Ethylhexylacrylat, Vinylacetat, Acrylsäure und Titanchelatester enthält.

5. Verfahren nach Anspruch 1, dadurch gekennzeichnet, daß das Polymermaterial nicht selbstvernetzendes Acrylatcopolymere aus 2-Ethylhexylacrylat, Vinylacetat und Acrylsäure enthält.

6. Verfahren nach Anspruch 1, dadurch gekennzeichnet, daß das Polymermaterial als Polymer auf Basis von Methacrylaten ein Copolymer auf Basis von Dimethylaminoethylmethacrylat und neutralen Methacrylsäureestern enthält.

7. Verfahren nach Anspruch 1, dadurch gekennzeichnet, daß das Polymermaterial als Ester des hydrierten Kolophoniums dessen Methylester enthält.

8. Verfahren nach Anspruch 1, dadurch gekennzeichnet, daß das Polymermaterial als Ester des hydrierten Kolophoniums dessen Glycerinester enthält.

9. Verfahren nach Anspruch 1, dadurch gekennzeichnet, daß die Reservoirschicht als Weichmacher Dioctylcyclohexan enthält.

10. Verfahren nach Anspruch 1, dadurch gekennzeichnet, daß die Reservoirschicht als Weichmacher Di-n-butyladipat enthält.

11. Verfahren nach Anspruch 1, dadurch gekennzeichnet, daß die Reservoirschicht als Weichmacher Triglyceride enthält.

12. Verfahren nach Anspruch 1, dadurch gekennzeichnet, daß die Reservoirschicht als Weichmacher Isopropylmyristat enthält.

13. Verfahren zur Herstellung eines transdermalen therapeutischen Systems nach einem der Ansprüche 1 bis 12, dadurch gekennzeichnet, daß die Bestandteile der Reservoirschicht in einem niedrig siedenden Lösemittel gelöst werden, das eine Trocknung bis zu einem maximalen Lösemittelrestgehalt von kleiner 0,4 Gew.-% ermöglicht.

14. Verfahren zur Herstellung eines transdermalen therapeutischen Systems nach Anspruch 13, dadurch gekennzeichnet, daß man ein Lösemittelgemisch aus Butanon und n-Heptan verwendet.

15. Träger für die Verwendung zur transdermalen Verabreichung von Physostigmin, bestehend aus einer wirkstoffundurchlässigen Rückschicht, einer haftklebenden Schicht, die zur Aufnahme des Wirkstoffs geeignet ist, sowie gegebenenfalls einer wiederablösbaren Schutzschicht, dadurch gekennzeichnet, daß die Reservoirschicht haftklebend ist und 10 bis 90 Gew.-% Polymermaterial sowie 0 bis 30 Gew.-% Weichmacher enthält.

## Claims (Claims for the following Contracting State(s): AT, BE, CH, DE, FR, GB, IT, LI, LU, NL, SE)

1. A transdermal therapeutical system for the administration of physostigmine to the skin, consisting of a cover layer which is impermeable to active substances, a pressure-sensitive adhesive reservoir layer, and optionally a removable protective layer, characterized in that the reservoir layer, starting from a solution in a low-boiling solvent or solvent mixture, comprises the following portions in homogeneous fine dispersion:
- 10 - 90%-wt polymeric material selected from the groups consisting of block copolymers on the basis of styrene and 1,3-dienes, polyisobutylene, polymers on the basis of acrylate and/or methacrylate and, optionally, as additives, esters of hydrogenated colophonium,
- 0 - 30%-wt softeners on the basis of hydrocarbons and/or esters, and
- 0.1 - 20%-wt physostigmine.

2. The transdermal therapeutical system according to claim 1, characterized in that the polymeric material comprises linear styrene-isoprene-styrene block copolymer.

3. The transdermal therapeutical system according to claim 1, characterized in that the polymeric material comprises linear styrene-butadiene-styrene block copolymer.

4. The transdermal therapeutical system according to claim 1, characterized in that the polymeric material comprises self-crosslinking acrylate copolymer of 2-ethylhexyl acrylate, vinyl acetate, acrylic acid and titanium chelate ester.

5. The transdermal therapeutical system according to claim 1, characterized in that the polymeric material comprises non-self-crosslinking acrylate copolymers of 2-ethylhexyl acrylate, vinyl acetate, and acrylic acid.

6. The transdermal therapeutical system according to claim 1, characterized in that the polymeric material comprises as polymer on the basis of methacrylates a copolymer on the basis of dimethylaminoethyl methacrylate and neutral methacrylic acid esters.

7. The transdermal therapeutical system according to claim 1, characterized in that the polymeric material comprises as ester of the hydrogenated colophonium the methylester thereof.

8. The transdermal therapeutical system according to claim 1, characterized in that the polymeric material comprises as ester of the hydrogenated colophonium the glycerol ester thereof.

9. The transdermal therapeutical system according to claim 1, characterized in that the reservoir layer comprises as softener dioctyl cyclohexane.

10. The transdermal therapeutical system according to claim 1, characterized in that the reservoir layer comprises as softener di-n-butyl adipate.

11. The transdermal therapeutical system according to claim 1, characterized in that the reservoir layer comprises as softener triglycerides.

12. The transdermal therapeutical system according to claim 1, characterized in that the reservoir layer comprises as softener isopropylmyristate.

13. A process for the production of a transdermal therapeutical system according to any one of claims 1 to 12, characterized in that the components of the reservoir layer are dissolved in a low-boiling solvent which makes possible drying up to a maximum residual solvent content of lesser than 0.4%-wt.

14. The process for the production of a transdermal therapeutical system according to claim 13, characterized in that a solvent mixture of butanone and n-heptane is used.

## Claims (Claims for the following Contracting State(s): ES, GR)

1. Process for the production of a transdermal therapeutical system for the administration of physostigmine to the skin, consisting of a cover layer which is impermeable to active substances, a pressure-sensitive adhesive reservoir layer, and optionally a removable protective layer, characterized in that the reservoir layer, starting from a solution in a low-boiling solvent or solvent mixture, comprises the following portions in homogeneous fine dispersion:
- 10 - 90%-wt polymeric material selected from the groups consisting of block copolymers on the basis of styrene and 1,3-dienes, polyisobutylene, polymers on the basis of acrylate and/or methacrylate and, optionally, as additives, esters of hydrogenated colophonium,
- 0 - 30%-wt softeners on the basis of hydrocarbons and/or esters, and
- 0.1 - 20%-wt physostigmine.

2. The process according to claim 1, characterized in that the polymeric material comprises linear styrene-isoprene-styrene block copolymer.

3. The process according to claim 1, characterized in that the polymeric material comprises linear styrene-butadiene-styrene block copolymer.

4. The process according to claim 1, characterized in that the polymeric material comprises self-crosslinking acrylate copolymer of 2-ethylhexyl acrylate, vinyl acetate, acrylic acid and titanium chelate ester.

5. The process according to claim 1, characterized in that the polymeric material comprises non-self-crosslinking acrylate copolymers of 2-ethylhexyl acrylate, vinyl acetate, and acrylic acid.

6. The process according to claim 1, characterized in that the polymeric material comprises as polymer on the basis of methacrylates a copolymer on the basis of dimethylaminoethyl methacrylate and neutral methacrylic acid esters.

7. The process according to claim 1, characterized in that the polymeric material comprises as ester of the hydrogenated colophonium the methylester thereof.

8. The process according to claim 1, characterized in that the polymeric material comprises as ester of the hydrogenated colophonium the glycerol ester thereof.

9. The process according to claim 1, characterized in that the reservoir layer comprises as softener dioctyl cyclohexane.

10. The process according to claim 1, characterized in that the reservoir layer comprises as softener di-n-butyl adipate.

11. The process according to claim 1, characterized in that the reservoir layer comprises as softener triglycerides.

12. The process according to claim 1, characterized in that the reservoir layer comprises as softener isopropylmyristate.

13. The process for the production of a transdermal therapeutical system according to any one of claims 1 to 12, characterized in that the components of the reservoir layer are dissolved in a low-boiling solvent which makes possible drying up to a maximum residual solvent content of lesser than 0.4%-wt.

14. The process for the production of a transdermal therapeutical system according to claim 13, characterized in that a solvent mixture of butanone and n-heptane is used.

15. Carrier to be used for the transdermal administration of physostigmin, consisting of a backing layer which is impermeable to active substances, a pressure-sensitive adhesive layer capable of absorbing the active substance, as well as, optionally, a removable protective layer, characterized in that the reservoir layer is pressure-sensitive adhesive and contains 10 - 90%-wt polymeric material as well as 0 - 30%-wt softener.

## Revendications (Revendications pour l'(les) Etat(s) contractant(s) suivant(s): AT, BE, CH, DE, FR, GB, IT, LI, LU, NL, SE)

1. Système thérapeutique, transdermique, destiné à l'administration de physostigmine à la peau, constitué d'une couche de couverture imperméable au principe actif, d'une couche réservoir collante et, éventuellement, d'une couche de protection amovible, caractérisé en ce que la couche réservoir, partant d'une solution dans un solvant ou un mélange de solvants à bas point d'ébullition, contient les parties suivantes en fine répartition homogène :
- 10 à 90% en poids d'une matière polymérique, choisie dans les groupes constitués de copolymères séquencés à base de styrène et de 1,3-diènes, de polyisobutylène, de polymères à base d'acrylate et/ou de méthacrylate et, éventuellement, à titre d'additifs, d'esters de la colophane hydrogénée,
- 0 à 30% en poids de plastifiants à base d'hydrocarbures et/ou d'esters et
- 0,1 à 20% en poids de physostigmine.

2. Système thérapeutique, transdermique, suivant la revendication 1, caractérisé en ce que la matière polymérique contient un copolymère séquencé de styrène, d' isoprène et de styrène, linéaire.

3. Système thérapeutique, transdermique, suivant la revendication 1, caractérisé en ce que la matière polymérique contient un copolymère séquencé de styrène, de butadiène et de styrène linéaire.

4. Système thérapeutique, transdermique, suivant la revendication 1, caractérisé en ce que la matière polymérique contient un copolymère d'acrylate autoréticulant, constitué d'acrylate de 2-éthylhexyle, d'acétate de vinyle, d'acide acrylique et d'un ester chélate de titane.

5. Système thérapeutique, transdermique, suivant la revendication 1, caractérisé en ce que la matière polymérique contient un copolymère d'acrylate non autoréticulant, constitué d'acrylate de 2-éthylhexyle, d'acétate de vinyle et d'acide acrylique.

6. Système thérapeutique, transdermique, suivant la revendication 1, caractérisé en ce que la matière polymérique contient, à titre de polymère à base de méthacrylates, un copolymère à base de méthacrylate de diméthylaminoéthyle et d'esters de l'acide méthacrylique neutres.

7. Système thérapeutique, transdermique, suivant la revendication 1, caractérisé en ce que la matière polymérique contient, à titre d'ester de la colophane hydrogénée, son ester méthylique.

8. Système thérapeutique, transdermique, suivant la revendication 1, caractérisé en ce que la matière polymérique contient, à titre d'ester de la colophane hydrogénée, son ester glycérinique.

9. Système thérapeutique, transdermique, suivant la revendication 1, caractérisé en ce que la couche réservoir contient du dioctylcyclohexane à titre de plastifiant.

10. Système thérapeutique, transdermique, suivant la revendication 1, caractérisé en ce que la couche réservoir contient de l'adipate de di-n-butyle à titre de plastifiant.

11. Système thérapeutique, transdermique, suivant la revendication 1, caractérisé en ce que la couche réservoir contient des triglycérides à titre de plastifiants.

12. Système thérapeutique, transdermique, suivant la revendication 1, caractérisé en ce que la couche réservoir contient du myristate d'isopropyle à titre de plastifiant.

13. Procédé de préparation d'un système thérapeutique, transdermique, suivant l'une quelconque des revendications 1 à 12, caractérisé en ce que l'on dissout les constituants de la couche réservoir dans un solvant à bas point d'ébullition, qui permet un séchage jusqu'à une teneur résiduelle maximale en solvant inférieure à 0,4% en poids.

14. Procédé de préparation d'un système thérapeutique, transdermique, suivant la revendication 13, caractérisé en ce que l'on utilise un mélange de solvants formé de butanone et de n-heptane.

## Revendications (Revendications pour l'(les) Etat(s) contractant(s) suivant(s): ES, GR)

1. Procédé de préparation d'un système thérapeutique, transdermique, destiné à l'administration de physostigmine à la peau, constitué d'une couche de couverture imperméable au principe actif, d'une couche réservoir collante et, éventuellement, d'une couche de protection amovible, caractérisé en ce que la couche réservoir, partant d'une solution dans un solvant ou un mélange de solvants à bas point d'ébullition, contient les parties suivantes en fine répartition homogène :
- 10 à 90% en poids d'une matière polymérique, choisie dans les groupes constitués de copolymères séquencés à base de styrène et de 1,3-diènes, de polyisobutylène, de polymères à base d'acrylate et/ou de méthacrylate et, éventuellement, à titre d'additifs, d'esters de la colophane hydrogénée,
- 0 à 30% en poids de plastifiants à base d'hydrocarbures et/ou d'esters et
- 0,1 à 20% en poids de physostigmine.

2. Procédé suivant la revendication 1, caractérisé en ce que la matière polymérique contient un copolymère séquencé de styrène, d'isoprène et de styrène, linéaire.

3. Procédé suivant la revendication 1, caractérisé en ce que la matière polymérique contient un copolymère séquencé de styrène, de butadiène et de styrène linéaire.

4. Procédé suivant la revendication 1, caractérisé en ce que la matière polymérique contient un copolymère d'acrylate autoréticulant, constitué d' acrylate de 2-éthylhexyle, d'acétate de vinyle, d'acide acrylique et d'un ester chélate de titane.

5. Procédé suivant la revendication 1, caractérisé en ce que la matière polymérique contient un copolymère d'acrylate non autoréticulant, constitué d'acrylate de 2-éthylhexyle, d'acétate de vinyle et d' acide acrylique.

6. Procédé suivant la revendication 1, caractérisé en ce que la matière polymérique contient, à titre de polymère à base de méthacrylates, un copolymère à base de méthacrylate de diméthylaminoéthyle et d'esters de l'acide méthacrylique neutres.

7. Procédé suivant la revendication 1, caractérisé en ce que la matière polymérique contient, à titre d'ester de la colophane hydrogénée, son ester méthylique.

8. Procédé suivant la revendication 1, caractérisé en ce que la matière polymérique contient, à titre d'ester de la colophane hydrogénée, son ester glycérinique.

9. Procédé suivant la revendication 1, caractérisé en ce que la couche réservoir contient du dioctylcyclohexane à titre de plastifiant.

10. Procédé suivant la revendication 1, caractérisé en ce que la couche réservoir contient de l'adipate de di-n-butyle à titre de plastifiant.

11. Procédé suivant la revendication 1, caractérisé en ce que la couche réservoir contient des triglycérides à titre de plastifiants.

12. Procédé suivant la revendication 1, caractérisé en ce que la couche réservoir contient du myristate d' isopropyle à titre de plastifiant.

13. Procédé de préparation d'un système thérapeutique, transdermique, suivant l'une quelconque des revendications 1 à 12, caractérisé en ce que l'on dissout les constituants de la couche réservoir dans un solvant à bas point d'ébullition, qui permet un séchage jusqu'à une teneur résiduelle maximale en solvant inférieure à 0,4% en poids.

14. Procédé de préparation d'un système thérapeutique, transdermique, suivant la revendication 13, caractérisé en ce que l'on utilise un mélange de solvants formé de butanone et de n-heptane.

15. Support pour l'utilisation en vue de l'administration transdermique de physostigmine, constitué d'une couche dorsale imperméable au principe actif, d'une couche collant par contact, qui convient à l'absorption du principe actif, comme éventuellement d'une couche de protection amovible, caractérisé en ce que la couche réservoir est collante par contact et contient 10 à 90% en poids d'une matière polymérique, ainsi que 0 à 30% en poids d'un plastifiant.
